# EUROPEAN PATENT APPLICATION

(11) **EP 4 647 500 A1**
(43) Date of publication of application: **12.11.2025**
(21) Application number: 24763217.7
(22) Date of filing: 29.02.2024
(51) Int. Cl.: C12P 13/04, A01N 57/20, C12N 9/10, C08K 5/5313

(54) **METHOD FOR DIRECTLY PREPARING L-GLUFOSINATE FROM L-HOMOSERINE**

(30) Priority: 01.03.2023 CN 202310184192
(71) Applicant: Hunan Lier Biotech Co., Ltd., Jia Shan Street Jinshi Hunan 415400 (CN)
(72) Inventor: LAI, Fan, Changde, Hunan 415400 (CN); REN, Jie, Changde, Hunan 415400 (CN); YANG, Pingping, Changde, Hunan 415400 (CN); FAN, Qian, Changde, Hunan 415400 (CN)
(74) Representative: Michalski Hüttermann & Partner Patentanwälte mbB
(86) International application number: PCT/CN2024/079261
(87) International publication number: WO 2024/179537

(57) **Abstract**

Provided is a process for preparing L-glufosinate by enzymatic catalysis reaction of homoserine and methylphosphinic acid. The process according to the present disclosure does not comprise a step of generating O-acyl -L-homoserine, and O-acyl-L-homoserine is not sued in the process.

## Description

### Technical Field

The present disclosure belongs to the field of biochemical engineering and specifically relates to a process for the direct enzymatic preparation of L-glufosinate using L-homoserine as a substrate.

### Background

Glufosinate (also known as 4-[hydroxyl(methyl) phosphono]-D,L- homoalanine) is the second best-selling herbicide to which genetically modified crops are tolerant in the world. Glufosinate is a broad-spectrum contact herbicide which inhibits the activity of L-glutamine synthase in plants, leading to disorder in nitrogen metabolism and ultimately killing the plants. As compared with glyphosate, glufosinate has significant advantages, such as wide application range, fast effecting, long-term effect, low toxicity, safety or the like. Therefore, the sales of glufosinate have grown rapidly, and there is a huge market and promising demand in the future.

However, the complex preparing process of glufosinate results in a high technical difficulty in production. High price prevents its rapid replacement of glyphosate. In addition, chemically synthesized glufosinate is a racemic mixture containing equal amounts of two optical isomers (D,L-glufosinate), in which only the L-configuration is physiologically active.

Recently, there have been numerous reports on the preparation of L-glufosinate from D,L-glufosinate. For example, D-glufosinate is oxidized to 2-carbonyl-4-(hydroxylmethylphosphono) butyric acid (PPO), which is then reduced or transaminated to generate L-glufosinate.

However, the process of chemically synthesizing D/L glufosinate and then converting D-glufosinate into L-glufosinate through enzymatic methods is complex and costly. Therefore, there is still a need of more efficient processes for synthesizing L-glufosinate.

WO 2022/207543 A1 reports a process using activated homoserine (O-acetyl homoserine or O-succinyl homoserine) and methylphosphinic acid or an ester thereof as substrates under enzymatic catalysis (hydrogen sulfide lyase or cystathionine γ synthase) to synthesize L-glufosinate or a phosphonate thereof.

However, this process requires the production (e.g., through fermentation) of O-acyl homoserine first, which is limited by i) accumulation of O-acyl homoserine; ii) instability of O-acyl homoserine (to acid, base, and heating); iii) inhibition of enzymatic activity by by-product organic acids; and iv) if the pH is adjusted in the reaction process, the production cost, process complexity and product separation difficulty will be increased.

Therefore, there is a need of developing a new process for preparing L-glufosinate, using a stable substrate which is easily obtainable, reducing product inhibition of the reaction, simplifying the process (including production and separation), and reducing costs.

### Summary

Surprisingly, the present inventors found that L-homoserine can react with methylphosphinic acid or an ester thereof under enzymatic catalysis to produce L-glufosinate or a phosphonate thereof and water (as shown in Formula I).

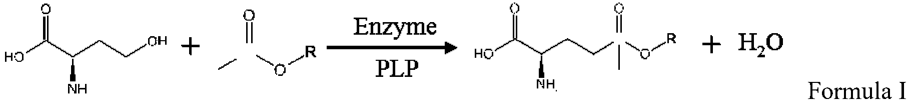

L-homoserine is a compound which is easier to obtain and more stable than O-acyl homoserine, and the reaction of the present disclosure does not produce an organic acid byproduct which inhibits enzymatic activity.

Accordingly, provided is a process for preparing L-glufosinate, comprising steps of:
a) providing a reaction medium, comprising L-homoserine or a salt, an ester, an amide or an anhydride thereof, methylphosphinic acid or an ester thereof, and an enzyme selected from the group consisting of a transferase which transfers an alkyl other than methyl or an aryl, ammonia lyase, and a lyase which catalyzes the cleavage of a carbon-sulfur bond;
b) incubating the reaction medium to produce a reaction product comprising L-glufosinate or a phosphonate thereof; and
   optionally,
c) recovering the L-glufosinate or the phosphonate thereof from the reaction medium.

In some embodiments, the enzyme is selected from the group consisting of enzymes with the EC numbers of EC 2.5.1.-, EC 4.3.1.- and EC 4.4.1.-.

In some embodiments, the reaction medium further comprises phosphopyridoxal or a salt thereof.

In some embodiments, the enzyme is selected from the group consisting of enzymes with the EC numbers of EC 2.5.1.47, EC 2.5.1.48, EC 2.5.1.49, EC 2.5.1.51, EC 2.5.1.52, EC 2.5.1.65, EC 2.5.1.76, EC 2.5.1.113, EC 2.5.1.134, EC 2.5.1.140, EC 2.5.1.144, EC 4.3.1.15, EC 4.3.1.17, EC 4.3.1.18, EC 4.3.1.19, EC 4.4.1.1, EC 4.4.1.2, EC 4.4.1.9, EC 4.4.1.10, EC 4.4.1.11, EC 4.4.1.13, EC 4.4.1.15, EC 4.4.1.16, EC 4.4.1.25, EC 4.4.1.28 and EC 4.4.1.35.

In some embodiments, the enzyme is derived from an organism selected from the group consisting of *Thermus thermophiles, Lysinibacillus sphaericus, Clostridioides difficile, Clostridium novyi, Geobacillus stearothermophilus, Thermotoga maritima, Pseudomonas aeruginosa, Nonlabens dokdonensis, Leucaena leucocephala, Aeropyrum pernix, Methanosarcina acetivorans, Mycobacterium tuberculosis, Bacillus subtilis, Staphylococcus aureus, Pseudomonas fluorescens, Escherichia coli, Candida maltosa, Streptomyces phaeochromogenes, Neurospora crassa, Spinacia oleracea, Cyanobacteria bacterium, Pseudomonas putida, Lactococcus lactis, Ruegeria pomeroyi, Methanococcus maripaludis* and *Camelina sativa.*

In some embodiments, the enzyme is selected from the group consisting of cysteine synthase derived from *Thermus thermophiles* (EC 2.5.1.47 - cysteine synthase), cystathionine γ-synthase derived from *Lysinibacillus sphaericus* (EC 2.5.1.48 - cystathionine gamma-synthase), O-acetyl homoserine aminocarboxypropyltransferase derived from *Clostridioides difficile* (EC 2.5.1.49 - O-acetylhomoserine aminocarboxypropyltransferase), O-acetyl homoserine aminocarboxypropyltransferase derived from *Clostridium novyi,* O-acetyl homoserine aminocarboxypropyltransferase derived from *Geobacillus stearothermophilus,* O-acetyl homoserine aminocarboxypropyltransferase derived from *Thermotoga maritima,* O-acetyl homoserine aminocarboxypropyltransferase derived from *Pseudomonas aeruginosa,* β pyrazolylalanine synthase derived from *Nonlabens dokdonensis* (EC 2.5.1.51 - beta-pyrazolylalanine synthase), L-mimosine synthase derived from *Leucaena leucocephala* (EC 2.5.1.52 - L-mimosine synthase), O-phosphoserine sulfhydrylase derived from *Aeropyrum pernix* (EC 2.5.1.65 - O-phosphoserine sulfhydrylase), cysteine synthase derived from *Methanosarcina acetivorans* (EC 2.5.1.76 - cysteate synthase), [CysO sulfur-carrier protein]-thiocarboxylate-dependent cysteine synthase derived from *Mycobacterium tuberculosis* (EC 2.5.1.113 - [CysO sulfur-carrier protein]-thiocarboxylate-dependent cysteine synthase), cystathionine β synthase derived from *Bacillus subtilis* (EC 2.5.1.134 - cystathionine beta-synthase), N-(2-amino-2-carboxyethyl)-L-glutamate synthase derived from *Staphylococcus aureus* (EC 2.5.1.140 - N-(2-amino-2-carboxyethyl)-L-glutamate synthase), S-sulfo-L-cysteine synthase derived from *Pseudomonas fluorescens* (EC 2.5.1.144 - S-sulfo-L-cysteine synthase), diaminopropionate ammonia-lyase derived from *Escherichia coli* (EC 4.3.1.15 - Diaminopropionate ammonia-lyase), L-serine ammonia-lyase derived from *Escherichia coli* 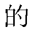 (EC 4.3.1.17 - L-serine ammonia-lyase), D-serine ammonia-lyase derived from *Escherichia coli* (EC 4.3.1.18 - D-serine ammonia-lyase), threonine ammonia-lyase derived from *Candida maltose* (EC 4.3.1.19 - threonine ammonia-lyase), cystathionine γ lyase derived from *Streptomyces phaeochromogenes* (EC 4.4.1.1 - cystathionine gamma-lyase), cystathionine γ lyase derived from *Neurospora crassa* (EC 4.4.1.1 - cystathionine gamma-lyase), homocysteine desulfhydrase derived from *Bacillus subtilis* 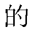 (EC 4.4.1.2 - homocysteine desulfhydrase), L-3-cyanoalanine synthase derived from *Spinacia oleracea* (EC 4.4.1.9 - L-3-cyanoalanine synthase), cysteine lyase derived from *Cyanobacteria bacterium* (EC 4.4.1.10 - cysteine lyase), methionine γ lyase derived from *Pseudomonas putida* (EC 4.4.1.11 - methionine gamma-lyase), cysteine-S-conjugate β lyase derived from *Lactococcus lactis* (EC 4.4.1.13 - cysteine-S-conjugate beta-lyase), D-cysteine desulfhydrase derived from *Escherichia coli* (EC 4.4.1.15 - D-cysteine desulfhydrase), selenocysteine lyase derived from *Escherichia coli* (EC 4.4.1.16 - selenocysteine lyase), L-cysteate sulfo-lyase derived from *Ruegeria pomeroyi* (EC 4.4.1.25 - L-cysteate sulfo-lyase), L-cysteine desulfidase derived from *Methanococcus maripaludis* (EC 4.4.1.28 - L-cysteine desulfidase) and L-cystine β lyase derived from *Camelina sativa* 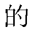 (EC 4.4.1.35 - L-cystine beta-lyase).

In some embodiments, the enzyme comprises an amino acid sequence selected from the group consisting of SEQ ID NO: 1-31.

In some embodiments, the process is a cell-free process.

In some embodiments, the reaction medium in step a) comprises a host cell expressing the enzyme. In some embodiments, the process further comprises a step of culturing the host cell.

In some embodiments, in step b), the reaction medium is incubated at 30-40°C, 32-39°C, 34-38°C, 36-38°C or 37°C.

### Brief Description of the Drawings

Figure 1 shows the LC-MS results of the reaction products catalyzed by purified GsMetY. A: the detection result of the reaction product sample, B: the detection result after addition of the internal standard.
Figure 2 shows the LC-MS results of the reaction products catalyzed by *Escherichia coli* expressing GsMetY. A: the detection result of the sample, B: the detection result after addition of the internal standard.
Figure 3 shows the LC-MS results of the reaction products catalyzed by *Escherichia coli* expressing SpCGL. A: the detection result of the sample, B: the detection result after addition of the internal standard.

### Detailed Description

Provided is a novel process for preparing L-glufosinate. Unless otherwise stated, the terms used herein have the general understanding of those skilled in the art.

It was reported in the prior art that 4-hydroxyl activated L-homoserine (such as O-acyl homoserine) can react with methylphosphinic acid or an ester thereof under enzymatic catalysis to produce L-glufosinate or a phosphonate ester thereof.

Surprisingly, the present inventors found that L-homoserine can react with methylphosphinic acid or an ester thereof under enzymatic catalysis to produce L-glufosinate or a phosphonate thereof and water (as shown in Formula I).

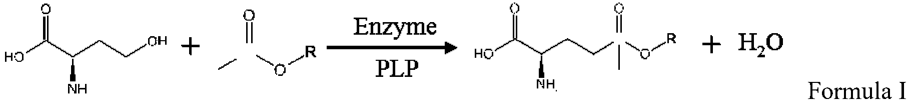

L-homoserine is a compound which is easier to obtain and more stable than O-acyl homoserine, and the reaction of Formula I does not produce an organic acid byproduct which inhibits enzymatic activity.

Accordingly, provided is a process for preparing L-glufosinate, comprising steps of:
a) providing a reaction medium, comprising a L-homoserine donor, methylphosphinic acid or an ester thereof, and an enzyme selected from the group consisting of a transferase which transfers an alkyl other than methyl or an aryl, an ammonia lyase, and a lyase which catalyzes the cleavage of a carbon-sulfur bond;
b) incubating the reaction medium to produce a reaction product comprising L-glufosinate or a phosphonate thereof; and
   optionally,
c) recovering the L-glufosinate or the phosphonate thereof from the reaction medium.

### I. Substrate

As used herein, "L-homoserine" refers to L-2-amino-4-hydroxyl-1-butanoic acid, with the molecular formula of C₄H₉NO₃. As used herein, a L-homoserine donor comprises but not limited to L-homoserine or a salt, an ester or an amide thereof, as well as an anhydride formed by L-homoserine with L-homoserine or another acid. The L-homoserine donor also comprises a derivative with one or two hydrogens substituted on its amino group. The ester of L-homoserine refers to a compound produced by the reaction of the carboxyl group of L-homoserine with an alcohol or a phenol. The amide of L-homoserine refers to a compound produced by the reaction of the carboxyl group of L-homoserine with an amine compound.

As used herein, the L-homoserine donor does not comprise a derivative where the hydrogen atom of 4-hydroxyl is substituted by acyl, i.e., O-acyl homoserine.

In step b), L-homoserine is the direct substrate of the enzyme. The process according to the present disclosure does not comprise a step of generating O-acyl-L-homoserine, and O-acyl-L-homoserine is not used in the process.

Methylphosphinic acid or an ester thereof is as shown in Formula II. 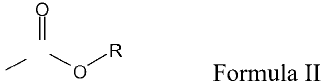 wherein, R is selected from the group consisting of hydrogen, alkyl, alkenyl, alkynyl, hydroxyalkyl and aryl.

In some embodiments, the reaction medium comprises methyl phosphinate. In some embodiments, the process further comprises a step of hydrolyzing L-glufosinate phosphonate.

### II. Enzyme

As mentioned above, the reaction of the present process is conducted under the catalysis of enzymes, i.e., an enzymatic reaction.

As used herein, the term "enzyme" refers to a protein or peptide which can specifically catalyze or promote a chemical or biochemical reaction. The enzyme according to the present disclosure may be an intact natural protein or polypeptide or a portion thereof with catalytic activity. The enzyme according to the present disclosure may also be a modified variant of a natural protein or polypeptide. The enzyme according to the present disclosure may be isolated from a living organism, or produced by recombination or synthesis. Those skilled in the art can obtain enzymes through conventional methods.

As used herein, the term "peptide" refers to a chain of at least two amino acids connected by peptide bonds. The term "polypeptide" can be used interchangeably with the term "protein" herein, referring to a chain containing ten or more amino acid residues. All peptide and polypeptide chemical formulae or sequences herein are written from left to right, indicating the direction from the amino terminal to the carboxyl terminal.

The term "amino acid" comprises naturally occurring amino acids and non-naturally occurring amino acids in proteins. The single letter and three letter names of naturally occurring amino acids in proteins are commonly used in the art, see Sambrook, et al. (Molecular Cloning: A Laboratory Manual, 2nd, ed. Cold Spring Harbor Laboratory, Cold Spring Harbor Laboratory Press, Cold Spring Harbor, N.Y., 1989).

| Amino Acid | Single letter | Three letter |
|---|---|---|
| Alanine | A | Ala |
| Arginine | R | Arg |
| Asparagine | N | Asn |
| Aspartate | D | Asp |
| Cysteine | C | Cys |
| Glutamine | Q | Gln |
| Glutamate | E | Glu |
| Glycine | G | Gly |
| Histidine | H | His |
| Isoleucine | I | Ile |
| Ieucine | L | Leu |
| Lysine | K | Lys |
| Methionine | M | Met |
| Phenylalanine | F | Phe |
| Proline | P | Pro |
| Serine | S | Ser |
| Threonine | T | Thr |
| Tryptophan | W | Trp |
| Tyrosine | Y | Tyr |
| Valine | V | Val |

As used herein, the term "modification" refers to any modification of a peptide composed of the polypeptide or its homologous sequence according to the present disclosure, including but not limited to substitution, deletion, insertion, and/or addition of one or more amino acids.

Some enzymes requires the participation of a coenzyme for the catalysis. The term "coenzyme" is a general term for a type of organic cofactors, which are essential factors for enzyme-catalyzed redox reactions, group transfer, and isomerization reactions. They are involved in the function of transferring electrons, atoms, or groups in enzyme-catalyzed reactions.

The present inventors found that the enzyme as used in the present disclosure only uses phosphopyridoxal as a coenzyme. Accordingly, in some embodiments, the reaction medium further comprises phosphopyridoxal or a salt thereof.

In some embodiments, the enzyme is selected from the group consisting of enzymes with the EC numbers of EC 2.5.1.-, EC 4.3.1.- and EC 4.4.1.-.

In some embodiments, the enzyme is selected from the group consisting of enzymes with the EC numbers of EC 2.5.1.47, EC 2.5.1.48, EC 2.5.1.49, EC 2.5.1.51, EC 2.5.1.52, EC 2.5.1.65, EC 2.5.1.76, EC 2.5.1.113, EC 2.5.1.134, EC 2.5.1.140, EC 2.5.1.144, EC 4.3.1.15, EC 4.3.1.17, EC 4.3.1.18, EC 4.3.1.19, EC 4.4.1.1, EC 4.4.1.2, EC 4.4.1.9, EC 4.4.1.10, EC 4.4.1.11, EC 4.4.1.13, EC 4.4.1.15, EC 4.4.1.16, EC 4.4.1.25, EC 4.4.1.28 and EC 4.4.1.35.

In some embodiments, the enzyme is selected from the group consisting of cysteine synthase (EC 2.5.1.47 - cysteine synthase), cystathionine γ-synthase (EC 2.5.1.48 - cystathionine gamma-synthase), O-acetyl homoserine aminocarboxypropyltransferase (EC 2.5.1.49 - O-acetylhomoserine aminocarboxypropyltransferase), βpyrazolylalanine synthase (EC 2.5.1.51 - beta-pyrazolylalanine synthase), L-mimosine synthase (EC 2.5.1.52 - L-mimosine synthase), O-phosphoserine sulfhydrylase (EC 2.5.1.65 - O-phosphoserine sulfhydrylase), cysteine synthase (EC 2.5.1.76 - cysteate synthase), [CysO sulfur-carrier protein]-thiocarboxylate-dependent cysteine synthase (EC 2.5.1.113 - [CysO sulfur-carrier protein]-thiocarboxylate-dependent cysteine synthase), cystathionine β synthase (EC 2.5.1.134 - cystathionine beta-synthase), N-(2-amino-2-carboxyethyl)-L-glutamate synthase (EC 2.5.1.140 - N-(2-amino-2-carboxyethyl)-L-glutamate synthase), S-sulfo-L-cysteine synthase (EC 2.5.1.144 - S-sulfo-L-cysteine synthase), diaminopropionate ammonia-lyase (EC 4.3.1.15 - Diaminopropionate ammonia-lyase), L-serine ammonia-lyase (EC 4.3.1.17 - L-serine ammonia-lyase), D-serine ammonia-lyase (EC 4.3.1.18 - D-serine ammonia-lyase), threonine ammonia-lyase (EC 4.3.1.19 - threonine ammonia-lyase), cystathionine γ lyase (EC 4.4.1.1 - cystathionine gamma-lyase), homocysteine desulfhydrase (EC 4.4.1.2 - homocysteine desulfhydrase), L-3-cyanoalanine synthase (EC 4.4.1.9 - L-3-cyanoalanine synthase), cysteine lyase (EC 4.4.1.10 - cysteine lyase), methionine γ lyase (EC 4.4.1.11 - methionine gamma-lyase), cysteine-S-conjugate β lyase (EC 4.4.1.13 - cysteine-S-conjugate beta-lyase), D-cysteine desulfhydrase (EC 4.4.1.15 - D-cysteine desulfhydrase), selenocysteine lyase (EC 4.4.1.16 - selenocysteine lyase), L-cysteate sulfo-lyase (EC 4.4.1.25 - L-cysteate sulfo-lyase), L-cysteine desulfidase (EC 4.4.1.28 - L-cysteine desulfidase) and L-cystine β lyase (EC 4.4.1.35 - L-cystine beta-lyase).

The enzyme according to the present disclosure may be derived from any suitable organism, including prokaryotes and eukaryotes, for example but not limited to archaea, actinomycetes, bacteria, fungi, animals, plants, algae, and yeast. In some embodiments, the enzyme is derived from an organism selected from the group consisting of *Thermus thermophiles, Lysinibacillus sphaericus*)*, Clostridioides difficile, Clostridium novyi, Geobacillus stearothermophilus, Thermotoga maritima, Pseudomonas aeruginosa, Nonlabens dokdonensis, Leucaena leucocephala, Aeropyrum pernix, Methanosarcina acetivorans, Mycobacterium tuberculosis, Bacillus subtilis, Staphylococcus aureus, Pseudomonas fluorescens, Escherichia coli, Candida maltosa, Streptomyces phaeochromogenes, Neurospora crassa, Spinacia oleracea, Cyanobacteria bacterium, Pseudomonas putida, Lactococcus lactis, Ruegeria pomeroyi, Methanococcus maripaludis* and *Camelina sativa.*

In some embodiments, the enzyme is selected from the group consisting of cysteine synthase derived from *Thermus thermophiles* (EC 2.5.1.47 - cysteine synthase), cystathionine γ-synthase derived from *Lysinibacillus sphaericus* (EC 2.5.1.48 - cystathionine gamma-synthase), O-acetyl homoserine aminocarboxypropyltransferase derived from *Clostridioides difficile* (EC 2.5.1.49 - O-acetylhomoserine aminocarboxypropyltransferase), O-acetyl homoserine aminocarboxypropyltransferase derived from *Clostridium novyi,* O-acetyl homoserine aminocarboxypropyltransferase derived from *Geobacillus stearothermophilus,* O-acetyl homoserine aminocarboxypropyltransferase derived from *Thermotoga maritima,* O-acetyl homoserine aminocarboxypropyltransferase derived from *Pseudomonas aeruginosa,* β pyrazolylalanine synthase derived from *Nonlabens dokdonensis* (EC 2.5.1.51 - beta-pyrazolylalanine synthase), L-mimosine synthase derived from *Leucaena leucocephala* (EC 2.5.1.52 - L-mimosine synthase), O-phosphoserine sulfhydrylase derived from *Aeropyrum pernix* (EC 2.5.1.65 - O-phosphoserine sulfhydrylase), cysteine synthase derived from *Methanosarcina acetivorans* (EC 2.5.1.76 - cysteate synthase), [CysO sulfur-carrier protein]-thiocarboxylate-dependent cysteine synthase derived from *Mycobacterium tuberculosis* (EC 2.5.1.113 - [CysO sulfur-carrier protein]-thiocarboxylate-dependent cysteine synthase), cystathionine β synthase derived from *Bacillus subtilis* (EC 2.5.1.134 - cystathionine beta-synthase), N-(2-amino-2-carboxyethyl)-L-glutamate synthase derived from *Staphylococcus aureus* (EC 2.5.1.140 - N-(2-amino-2-carboxyethyl)-L-glutamate synthase), S-sulfo-L-cysteine synthase derived from *Pseudomonas fluorescens* (EC 2.5.1.144 - S-sulfo-L-cysteine synthase), diaminopropionate ammonia-lyase derived from *Escherichia coli* (EC 4.3.1.15 - Diaminopropionate ammonia-lyase), L-serine ammonia-lyase derived from *Escherichia coli* (EC 4.3.1.17 - L-serine ammonia-lyase), D-serine ammonia-lyase derived from *Escherichia coli* (EC 4.3.1.18 - D-serine ammonia-lyase), threonine ammonia-lyase derived from *Candida maltose* (EC 4.3.1.19 - threonine ammonia-lyase), cystathionine γ lyase derived from *Streptomyces phaeochromogenes* 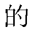 (EC 4.4.1.1 - cystathionine gamma-lyase), cystathionine γ lyase derived from *Neurospora crassa* (EC 4.4.1.1 - cystathionine gamma-lyase), homocysteine desulfhydrase derived from *Bacillus subtilis* (EC 4.4.1.2 - homocysteine desulfhydrase), L-3-cyanoalanine synthase derived from *Spinacia oleracea* (EC 4.4.1.9 - L-3-cyanoalanine synthase), cysteine lyase derived from *Cyanobacteria bacterium* (EC 4.4.1.10 - cysteine lyase), methionine γ lyase derived from *Pseudomonas putida* (EC 4.4.1.11 - methionine gamma-lyase), cysteine-S-conjugate β lyase derived from *Lactococcus lactis* (EC 4.4.1.13 - cysteine-S-conjugate beta-lyase), D-cysteine desulfhydrase derived from *Escherichia coli* (EC 4.4.1.15 - D-cysteine desulfhydrase), selenocysteine lyase derived from *Escherichia coli* (EC 4.4.1.16 - selenocysteine lyase), L-cysteate sulfo-lyase derived from *Ruegeria pomeroyi* (EC 4.4.1.25 - L-cysteate sulfo-lyase), L-cysteine desulfidase derived from *Methanococcus maripaludis* (EC 4.4.1.28 - L-cysteine desulfidase) and L-cystine β lyase derived from *Camelina sativa* (EC 4.4.1.35 - L-cystine beta-lyase). In some embodiments, the enzyme is O-acetyl homoserine aminocarboxypropyltransferase derived from *Clostridium novyi.*

In some embodiments, the enzyme comprises an amino acid sequence selected from the group consisting of SEQ ID NO: 1-31. In some embodiments, the enzyme is a modified variant.

As used herein, the term "variant" refers to a peptide or nucleic acid which has a certain sequence identity compared to the amino acid or nucleotide sequence of a given peptide or nucleic acid.

For the present disclosure, to determine the percentage of identity between two amino acid sequences or two nucleic acid sequences, sequences are aligned for optimal comparison (for example, a gap may be introduced in the first amino acid or nucleic acid sequence for optimal alignment with the second amino acid or nucleic acid sequence). Then the amino acid residues or nucleotides at the corresponding amino acid or nucleotide positions are compared. When the position in the first sequence is occupied by the same amino acid residue or nucleotide in the corresponding position in the second sequence, these molecules are identical at that position. The identity percentage between two sequences is a function of the number of identical positions shared by the sequence (i.e., identity percentage = number of identical positions / total number of positions (i.e., overlapping positions) × 100). Preferably, these two sequences are of the same length.

Those skilled in the art know that various computer programs may be used to determine the identity between two sequences.

"Amino acid identity percentage" or "amino acid sequence identity percentage" refers to comparison of amino acids of two polypeptides, and when the optimal comparison is made, the two polypeptides have approximately the specified percentage of identical amino acids. For example, "95% amino acid identity" means that two polypeptides have 95% identical amino acids when the two polypeptides are compared for amino acids at an optimal alignment.

In some embodiments, the enzyme comprises an amino acid sequence having at least 65% or 70%, preferably at least 75% or 80%, more preferably at least 85% or 90%, particularly preferably at least 94%, 95%, 96%, 97%, 98% or 99%, most preferably at least 99.1%, 99.2%, 99.3%, 99.4%, 99.5%, 99.6%, 99.7%, 99.8% or 99.9% sequence identity with an amino acid sequence selected from the group consisting of SEQ ID NO: 1-31, and the enzyme has the activity of catalyzing the reaction of Formula I.

In some embodiments, the amino acid sequence of the enzyme differs from the amino acid sequence selected from the group consisting of SEQ ID NO: 1-31 in having a substitution, deletion, insertion, and/or addition of one or more amino acids, and the enzyme has the activity of catalyzing the reaction of Formula I. In some embodiments, the amino acid sequence of the enzyme differs from the amino acid sequence selected from the group consisting of SEQ ID NO: 1-31 in having a conserved substitution of one or more amino acids, and the enzyme has the activity of catalyzing the reaction of Formula I. In some embodiments, the amino acid sequence of the enzyme differs from the amino acid sequence selected from the group consisting of SEQ ID NO: 1-31 in having an insertion or a deletion of one or more amino acids, and the enzyme has the activity of catalyzing the reaction of Formula I.

The term "conserved substitution", also known as substitution with "homologous" amino acid residue, refers to the substitution where an amino acid residue is replaced with another amino acid residue having a similar side chain, such as amino acids with basic side chain (such as lysine, arginine and histidine), amino acids with acidic side chain (such as aspartate, glutamate), amino acids with uncharged polar side chain (such as glycine, asparagine, glutamine, serine, threonine, tyrosine, cysteine), amino acids with nonpolar side chain (such as alanine, valine, leucine, isoleucine, proline, phenylalanine, methionine, tryptophan), amino acids with β-branched side chain (such as threonine, valine, isoleucine) and amino acids with aromatic side chain (such as tyrosine, phenylalanine, tryptophan, histidine).

The conserved amino acid substitution usually has the minimal influence on the activity of the resulted protein. Such substitution is described below. The conserved substitution is the replacement of an amino acid with another amino acid having similar size, hydrophobicity, charge, polarity, spatial characteristics, aromaticity, or the like. When it is desired to finely regulate the properties of a protein, the substitution is usually conserved.

As used herein, "homologous" amino acid residues refer to amino acid residues with similar chemical properties, which involve hydrophobicity, charge, polarity, spatial characteristics, aromatic characteristics, or the like. Examples of amino acids which are homologous to each other comprise positively charged lysine, arginine, histidine; negatively charged glutamate, aspartate; hydrophobic glycine, alanine, valine, leucine, isoleucine, proline, phenylalanine; polar serine, threonine, cysteine, methionine, tryptophan, tyrosine, asparagine, glutamine; aromatic phenylalanine, tyrosine, tryptophan; serine and threonine with chemically similar side chain groups, or glutamine and asparagine, or leucine and isoleucine.

Examples of conservative amino acid substitutions in proteins include: Ser for Ala, Lys for Arg, Gln or His for Asn, Glu for Asp, Ser for Cys, Asn for Gln, Asp for Glu, Pro for Gly, Asn or Gln for His, Leu or Val for Ile, Ile or Val for Leu, Arg or Gln for Lys, Leu or Ile for Met, Met, Leu or Tyr for Phe, Thr for Ser, Ser for Thr, Tyr for Trp, Trp or Phe for Tyr, and Ile or Leu for Val.

### III. Preparation conditions

The enzyme according to the present disclosure can be provided in the form of an isolated polypeptide.

In some embodiments, the process is a cell-free process, that is, the reaction medium in step a) comprises an isolated enzyme. The enzyme may be produced by recombination or synthesis. In some embodiments, the reaction medium is an aqueous medium, preferably an aqueous buffer, for example a phosphate buffer, such as PBS.

In some embodiments, the reaction medium in step a) comprise a host cell expressing the enzyme. In some embodiments, the process according to the present disclosure further comprises a step of culturing the host cell. In some embodiments, the reaction medium comprises a culture medium for culturing the host cell. In some embodiments, the reaction medium is an aqueous medium, preferably an aqueous buffer solution, for example a phosphate buffer solution, such as PBS.

In some embodiments, the host cell comprises an expression vector encoding the enzyme. In some embodiments, the host cell is *Escherichia coli,* for example *Escherichia coli* BL21(DE3). In some embodiments, the expression vector is a prokaryotic expression vector, for example pET-29b(+).

In some embodiments, in step b), the reaction medium is incubated at 30-40°C, 32-39°C, 34-38°C, 36-38°C or 37°C.

In some embodiments, the reaction medium has a PH of about 5-about 8, preferably about 6 - about 7.8, for example 7.5.

### Examples

Through the following examples, those skilled in the art will understand the present disclosure more clearly. It should be noted that the Examples are provided for illustrative purpose only rather than limitation to the scope of the present disclosure.

### Example 1 Materials and methods

Unless otherwise stated, the experimental methods used herein are conventional, and specific gene cloning procedures can be found in Sambrook et al., 1989 above.

### i) Reagents:

Isopropyl-β-D-thiogalactoside (IPTG) was purchased from Beyotime Biotechnology Co., Ltd.; L-homoserine was purchased from Jiangsu Aikon Biomedical Research and Development Co., Ltd.; Butyl methylphosphinate was synthesized in house according to the process reported in CN106674275B; L-glufosinate, phosphopyridoxal (PLP), and acetonitrile were purchased from Shanghai Aladdin Biochemical Technology Co., Ltd.; and Triton X100 was purchased from Sangon Biotechnology (Shanghai) Co., Ltd.

### ii) Vectors and strains:

- The expression vector used in the Examples was pET-29b(+), purchased from Sangon Biotechnology (Shanghai) Co., Ltd.
- The host cell used in the Example was *Escherichia coli* BL21(DE3), purchased from Tiangen Biochemical Technology (Beijing) Co., Ltd.

### iii) Gene cloning

The nucleic acid sequences encoding each enzyme in Table 1 were provided to Sangon Biotechnology (Shanghai) Co., Ltd. for synthesis, obtaining pET29b(+) expression vectors containing the nucleic acid sequences encoding each enzyme.

**Table 1**

| Enzyme | EC No. | Species | NCBI-Protein ID | Amino Acid Sequence |
|---|---|---|---|---|
| cysteine synthase | EC 2.5.1.47 | *Thermus thermophilus* | BAD70170 | SEQ ID NO: 1 |
| cystathionine γ-synthase | EC 2.5.1.48 | *Lysinibacillus sphaericus* | ACA40889 | SEQ ID NO: 2 |
| O-acetyl homoserine aminocarboxypropyltransferase | EC 2.5.1.49 | *Clostridioides difficile* | APU51341 | SEQ ID NO: 3 |
| O-acetyl homoserine aminocarboxypropyltransferase | EC 2.5.1.49 | *Clostridium novyi* | WP_011721301 | SEQ ID NO: 4 |
| O-acetyl homoserine aminocarboxypropyltransferase | EC 2.5.1.49 | *Geobacillus stearothermophilus* | ALA70660 | SEQ ID NO: 5 |
| O-acetyl homoserine aminocarboxypropyltransferase | EC 2.5.1.49 | *Thermotoga maritima* | WP_00408071 0 | SEQ ID NO: 6 |
| O-acetyl homoserine aminocarboxypropyltransferase | EC 2.5.1.49 | *Pseudomonas aeruginosa* | NP_253712 | SEQ ID NO: 7 |
| β pyrazolylalanine synthase | EC 2.5.1.51 | *Nonlabens dokdonensis* | BAA05965 | SEQ ID NO: 8 |
| L-mimosine synthase | EC 2.5.1.52 | *Leucaena leucocephala* | AHG97874 | SEQ ID NO: 9 |
| O-phosphoserine sulfhydrylase | EC 2.5.1.65 | *Aeropyrum pernix* | BAA80586 | SEQ ID NO: 10 |
| cysteine synthase | EC 2.5.1.76 | *Methanosarcina acetivorans* | AAM06667 | SEQ ID NO: 11 |
| [CysO sulfur-carrier protein]-thiocarboxylate-dependent cysteine synthase | EC 2.5.1.113 | *Mycobacterium tuberculosis* | NP_215852 | SEQ ID NO: 12 |
| cystathionine β synthase | EC 2.5.1.134 | *Bacillus subtilis* | NP_390604 | SEQ ID NO: 13 |
| N-(2-amino-2-carboxyethyl)-L-gluta mate synthase | EC 2.5.1.140 | *Staphylococcus aureus* | BAF66332 | SEQ ID NO: 14 |
| S-sulfo-L-cysteine synthase | EC 2.5.1.144 | *Pseudomonas fluorescens* | AIG05382 | SEQ ID NO: 15 |
| diaminopropionate ammonia-lyase | EC 4.3.1.15 | *Escherichia coli* | BAE76937 | SEQ ID NO: 16 |
| L-serine ammonia-lyase | EC 4.3.1.17 | *Escherichia coli* | BAA15621 | SEQ ID NO: 17 |
| D-serine ammonia-lyase | EC 4.3.1.18 | *Escherichia coli* | BAA16229 | SEQ ID NO: 18 |
| threonine ammonia-lyase | EC 4.3.1.19 | *Candida maltosa* | EMG50701 | SEQ ID NO: 19 |
| cystathionine γ lyase | EC 4.4.1.1 | *Streptomyces phaeochromogenes* | WP_26676142 6 | SEQ ID NO: 20 |
| cystathionine γ lyase | EC 4.4.1.1 | *Neurospora crassa* | AF401238_1 | SEQ ID NO: 21 |
| homocysteine desulfhydrase | EC 4.4.1.2 | *Bacillus subtilis* | NP_390603 | SEQ ID NO: 22 |
| L-3-cyanoalanine synthase | EC 4.4.1.9 | *Spinacia oleracea* | BAA07177.1 | SEQ ID NO: 23 |
| cysteine lyase | EC 4.4.1.10 | *Cyanobacteria bacterium* | HAA27123 | SEQ ID NO: 24 |
| methionine γ lyase | EC 4.4.1.11 | *Pseudomonas putida* | AAN66932 | SEQ ID NO: 25 |
| cysteine-S-conjugate β lyase | EC 4.4.1.13 | *Lactococcus lactis* | ABJ72384 | SEQ ID NO: 26 |
| D-cysteine desulfhydrase | EC 4.4.1.15 | *Escherichia coli* | BAA15739 | SEQ ID NO: 27 |
| selenocysteine lyase | EC 4.4.1.16 | *Escherichia coli* | BAA15457 | SEQ ID NO: 28 |
| L-cysteate sulfo-lyase | EC 4.4.1.25 | *Ruegeria pomeroyi* | AAV97294 | SEQ ID NO: 29 |
| L-cysteine desulfidase | EC 4.4.1.28 | *Methanococcus maripaludis* | CAF31024 | SEQ ID NO: 30 |
| L-cystine β lyase | EC 4.4.1.35 | *Camelina sativa* | XP_010509309 | SEQ ID NO: 31 |

### iv) Preparation of recombinant host cells

The expression vectors were transformed into *Escherichia coli* BL21(DE3) competent cells, respectively, which were spread om LB agar medium (containing 50mg/L kanamycin), incubated overnight at 37°C. Then single colonies were selected and cultured in LB broth (containing 50mg/L kanamycin) and subjected to sequencing to verify the correctness of the synthesized sequences. The verified clones were stored at -80°C for subsequent experiments.

### v) Protein expression and preparation of whole cell catalysts:

The stored clones were activated on LB agar medium. Then, the single colonies were inoculated in LB broth c(containing 50mg/L kanamycin) and incubated at 37°C for 12 h with shaking. 400 µL of culture was transferred into 20 mL of fresh LB liquid medium (containing 50mg/L kanamycin) and incubated at 37°C with shaking until the OD600 reached about 0.6. IPTG (final concentration of 0.4mM) was added and incubation was conducted at 25°C for 16 h to induce protein expression.

After incubation, the cultures were centrifuged at 4000g for 10 min at 4°C, the supernatants were discarded, and the *Escherichia coli* cells were collected. The collected *Escherichia coli* cells were resuspended in precooled 15 mL of 50 mM PBS, pH 7.0 to give *Escherichia coli* suspension containing the recombinant enzyme as whole cell catalysts.

### vi) Preparation of enzymes

7.5 mL of *Escherichia coli* suspension prepared in v) was taken and the *Escherichia coli* cells were sonicated at 4°C. The cell fragmentation solutions were centrifuged at 4°C at 6000g for 15 min to remove the precipitates, so as to obtain the resultant supernatants as crude enzyme solutions containing the recombinant enzymes. The crude enzyme solutions were purified using AKTA protein purification system to obtain the purified enzymes.

### vii) Catalyzing reaction with purified enzymes

Butyl methylphosphinate and PLP were added to a solution of L-homoserine in Tris (20mM), and the solution was adjusted to pH 7.4 with hydrochloric acid. In the solution, the final concentration of L-homoserine was 84mM, the final concentration of butyl methylphosphinate was 168mM, and the final concentration of PLP was 0.3mM. Purified enzyme as prepared in (vi) was added to the above solution and the final concentration was 0.5 mg/mL. The system was shaken on an oscillator at 37°C for 12 h (220 rpm). The sample was taken and hydrolyzed in a 100°C metal bath with 6 N HCl for 2 h, and the pH value was adjusted to 7.0 after hydrolysis. LC-MS was used to detect the hydrolyzed sample and 1 mM L-glufosinate was added as the internal standard sample. The peak with molecular weight of 182 under positive mode was extracted, to determine the generation of L-glufosinate.

### viii) Whole cell catalytic reaction

Butyl methylphosphinate, PLP and Triton X-100 were added to a solution of L-homoserine in Tris (20mM) successively, and the solution was adjusted to pH 7.4 with hydrochloric acid. In the solution, the final concentration of L-homoserine was 84mM, the final concentration of butyl methylphosphinate was 168mM, the final concentration of PLP was 0.3 mM, and the final concentration of Triton X-100 is 0.2%. Whole cells prepared according to the procedures in (v) were added to the above solutions respectively, and the final concentrations of the whole cells were 30 OD₆₀₀. At 37°C, the system was shaken on an oscillator for 12 h (220 rpm). The sample was taken and hydrolyzed in a 100°C metal bath with 6 N HCl for 2 h, and the pH value was adjusted to 7.0 after hydrolysis. LC-MS was used to detect the hydrolyzed sample and 1 mM L-glufosinate was added as the internal standard sample. The peak with molecular weight of 182 under positive mode was extracted, to determine the generation of L-glufosinate.

### Example 2. Preparation and detection of MetY (GsMetY) from Bacillus stearothermophilus

The clone expressing GsMetY (SEQ ID NO: 5) was activated, purified enzyme was prepared, and L-glufosinate produced by catalysis of the purified enzyme was detected according to the procedures in Example 1.

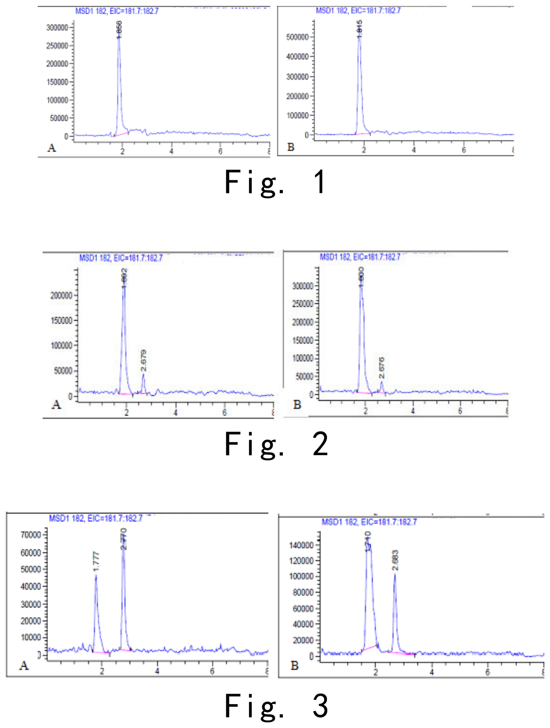

According to LC-MS detection, a peak with a molecular weight of 182 was extracted from the reaction product sample catalyzed by GsMetY (see Fig. 1A); only one peak with a molecular weight of 182 was extracted from the sample added with 1 mM L-glufosinate as internal standard, and the peak height increased (see Fig. 1B), indicating that the reaction product sample contained L-glufosinate, that is, L-glufosinate was produced by catalysis of GsMetY purified enzyme.

### Example 3. Preparation and detection of whole cell expressing GsMetY

The clone expressing GsMetY (SEQ ID NO: 5) was activated, whole cell was prepared, and L-glufosinate produced by catalysis of the whole cell was determined according to the procedures in Example 1.

According to LC-MS detection, a peak with a molecular weight of 182 was extracted from the reaction product sample catalyzed by whole cell expressing GsMetY (see Fig. 2A); no new peak was detected in the sample added with 1 mM L-glufosinate as internal standard, and the peak height and area near the retention time of 1.8 min increased (see Fig. 2B), indicating that the reaction product sample contained L-glufosinate, that is, L-glufosinate was produced by catalysis of whole cell expressing GsMetY.

### Example 4. Preparation and detection of whole cell expressing CGL(SpCGL) from Streptomyces phaeochromogenes

The clone expressing SpCGL (SEQ ID NO: 20) was activated, whole cell was prepared according to the procedures in Example 1, and L-glufosinate produced by catalysis of the whole cell was determined.

According to LC-MS detection, a peak with a molecular weight of 182 was extracted from the reaction product sample catalyzed by whole cell expressing SpCGL (see Fig. 3A); no new peak was detected in the sample added with 1 mM L-glufosinate as internal standard, and the peak height and area near the retention time of 1.8 min increased (see Fig. 3B), indicating that the reaction product sample contained L-glufosinate, that is, L-glufosinate was produced by catalysis of whole cell expressing SpCGL.

### Example 5. Production of L-glufosinate with Escherichia coli cells expressing foreign enzymes

This Example was carried out to determine whether the *Escherichia coli* cells expressing foreign enzymes can catalyze the conversion of L-homoserine to L-glufosinate.

As described in Example 1, recombinant *Escherichia coli* cells expressing each of the enzymes in Table 1 were prepared, and tested for the production of L-glufosinate catalyzed by the whole cells.

As shown in Table 2, the recombinant *Escherichia coli* cells expressing any of the enzymes of SEQ ID NOs: 1-31 can catalyze the conversion of L-homoserine to L-glufosinate, where the catalyzing ability was shown with ★, more ★ indicating stronger catalyzing ability.

**Table 2.**

| Enzyme | EC No. | Species | Amino Acid Sequence | Catalyzing ability |
|---|---|---|---|---|
| cysteine synthase | EC 2.5.1.47 | *Thermus thermophilus* | SEQ ID NO: 1 | ★ |
| cystathionine γ-synthase | EC 2.5.1.48 | *Lysinibacillus sphaericus* | SEQ ID NO: 2 | ★★ |
| O-acetyl homoserine aminocarboxypropyltransferase | EC 2.5.1.49 | *Clostridioides difficile* | SEQ ID NO: 3 | ★★★ |
| O-acetyl homoserine aminocarboxypropyltransferase | EC 2.5.1.49 | *Clostridium novyi* | SEQ ID NO: 4 | ★★★ |
| O-acetyl homoserine aminocarboxypropyltransferase | EC 2.5.1.49 | *Geobacillus stearothermophilus* | SEQ ID NO: 5 | ★★★★ |
| O-acetyl homoserine aminocarboxypropyltransferase | EC 2.5.1.49 | *Thermotoga maritima* | SEQ ID NO: 6 | ★★ |
| O-acetyl homoserine aminocarboxypropyltransferase | EC 2.5.1.49 | *Pseudomonas aeruginosa* | SEQ ID NO: 7 | ★ |
| β pyrazolylalanine synthase | EC 2.5.1.51 | *Nonlabens dokdonensis* | SEQ ID NO: 8 | ★ |
| L-mimosine synthase | EC 2.5.1.52 | *Leucaena leucocephala* | SEQ ID NO: 9 | ★ |
| O-phosphoserine sulfhydrylase | EC 2.5.1.65 | *Aeropyrum pernix* | SEQ ID NO: 10 | ★★ |
| cysteine synthase | EC 2.5.1.76 | *Methanosarcina acetivorans* | SEQ ID NO: 11 | ★ |
| [CysO sulfur-carrier protein]-thiocarboxylate-dependent cysteine synthase | EC 2.5.1.113 | *Mycobacterium tuberculosis* | SEQ ID NO: 12 | ★ |
| cystathionine β synthase | EC 2.5.1.134 | *Bacillus subtilis* | SEQ ID NO: 13 | ★★ |
| N-(2-amino-2-carboxyethyl)-L-gluta mate synthase | EC 2.5.1.140 | *Staphylococcus aureus* | SEQ ID NO: 14 | ★ |
| S-sulfo-L-cysteine synthase | EC 2.5.1.144 | *Pseudomonas fluorescens* | SEQ ID NO: 15 | ★★ |
| diaminopropionate ammonia-lyase | EC 4.3.1.15 | *Escherichia coli* | SEQ ID NO: 16 | ★ |
| L-serine ammonia-lyase | EC 4.3.1.17 | *Escherichia coli* | SEQ ID NO: 17 | ★ |
| D-serine ammonia-lyase | EC 4.3.1.18 | *Escherichia coli* | SEQ ID NO: 18 | ★ |
| threonine ammonia-lyase | EC 4.3.1.19 | *Candida maltosa* | SEQ ID NO: 19 | ★ |
| cystathionine γ lyase | EC 4.4.1.1 | *Streptomyces phaeochromogenes* | SEQ ID NO: 20 | ★★ |
| cystathionine γ lyase | EC 4.4.1.1 | *Neurospora crassa* | SEQ ID NO: 21 | ★★ |
| homocysteine desulfhydrase | EC 4.4.1.2 | *Bacillus subtilis* | SEQ ID NO: 22 | ★ |
| L-3-cyanoalanine synthase | EC 4.4.1.9 | *Spinacia oleracea* | SEQ ID NO: 23 | ★ |
| cysteine lyase | EC 4.4.1.10 | *Cyanobacteria bacterium* | SEQ ID NO: 24 | ★ |
| methionine γ lyase | EC 4.4.1.11 | *Pseudomonas putida* | SEQ ID NO: 25 | ★★★★ |
| cysteine-S-conjugate β lyase | EC 4.4.1.13 | *Lactococcus lactis* | SEQ ID NO: 26 | ★★ |
| D-cysteine desulfhydrase | EC 4.4.1.15 | *Escherichia coli* | SEQ ID NO: 27 | ★ |
| selenocysteine lyase | EC 4.4.1.16 | *Escherichia coli* | SEQ ID NO: 28 | ★ |
| L-cysteate sulfo-lyase | EC 4.4.1.25 | *Ruegeria pomeroyi* | SEQ ID NO: 29 | ★ |
| L-cysteine desulfidase | EC 4.4.1.28 | *Methanococcus maripaludis* | SEQ ID NO: 30 | ★ |
| L-cystine β lyase | EC 4.4.1.35 | *Camelina sativa* | SEQ ID NO: 31 | ★ |

Sequences

SEQ ID NO: 1
MRVEGAIGKTPVVRLAKVVEPDMAEVWVKLEGLNPGGSIKDRPAWYMIKDAEE
RGILRPGSGQVIVEPTSGNTGIGLAMIAASRGYRLILTMPAQMSEERKRVLKAFGA
ELVLTDPERRMLAAREEALRLKEELGAFMPDQFKNPANVRAHYETTGPELYEALE
GRIDAFVYGSGTGGTITGVGRYLKERIPHVKVIAVEPARSNVLSGGKMGQHGFQG
MGPGFIPENLDLSLLDGVIQVWEEDAFPLARRLAREEGLFLGMSSGGIVWAALQV
ARELGPGKRVACISPDGGWKYLSTPLYAEP

SEQ ID NO: 2
MTLNRSIETKLVQLGNLSDPTTGAVSPPIHLSTAYKHAGIGESTGFDYTRTKNPTRA
LLEAGFADLEGGDMGFACSSGMAAIQLVLSIFKPGDELVVPDDLYGGTYRLFNFFQ
ETYNIKPVYSKFESVEQVEALINEHTRALFIETPTNPLMQEFDLQVYAELAHKHGA
LLIVDNTFYTPYFQRPIDLGADIVLHSATKYIGGHNDVLAGIVVAKGTELAERIGFI
HNGSGMVLGAMDSWLLIRGLKTMHLRLKQHDANAKAIAAYLEEEALVTDVLYT
GKGGMLSFRLQKPEWIDPFLRNLKLITFAESLGGVESFITYPATQTHADMPYEERV
ERGVCDRLLRFSVGIEEAEDLIADLRQVFDILRKEA

SEQ ID NO: 3
MDNKETICVQGNYKPGNGEPRVLPLYQSTTFKYSSIDQLAELFDLKVDGHIYSRIS
NPTIQAFEEKISLLEGGVSSVAVSSGQSANMLAVLNICKSGDSILCSSKVYGGTFNL
LGPSLKKFGIDLISFDLDSSEDEIVELAKENTKVVFAETLANPTLEVIDFEKIANVAK
RINVPFIVDNSLASPVLCNPLKYGANIVTHSTTKYLDGHASSVGGIIVDGGNFNWD
NGKFPELVEPDPTYHGISYTQKFGNAAYATKARVQLLRDYGNCLSPFNAYLTNLN
VETLHLRMERHSENALKIARFLEKHENVDWINYPGLEDNKYYENAKKYLSRGCS
GVLSFGVRGGLENAKKFVEKLQIASLVTHVSDVRTCVIHPASTTHRQLTEEQLIAS
GVLPSLIRLSVGIENVEDLIADLNQALNF

SEQ ID NO: 4
MNNWKKGTICIQGGYTPKSGEPRVLPIYQSTTYKYNDPDEVAALFDLKAEGHMY
SRISNPTVAAFEEKIAALEGGVGALAVASGQSASTLSILNVCKSGEHVVAASTLYGG
TYSLFSTTLKKFGIDVTFVDPEATEEEILKNCRENTKAVFGETIGNPGLNVLDFEKF
SNVAKKIKAPFIVDNTIATPYLCNPLKLGANVVVHSATKYIDGHATTVGGVIIDGG
NFDWNNGRFPDFTEPDPSYHGIKYTETFGDSAYIVKARVQLLRDLGVCTSPFNAFL
FSLGLETLHLRMERHSDNAIKLGRFLEKHSKVSWVSYPLLESHSTYETAKKYLPK
GASGILTFGVKGGVEAGKQFIRNLKLAALVVHLGDARTSVLHPASTTHRQLTEEEQ
LSAGVTPDLIRVSVGIEDIEDIIEDFDQALNNIK

SEQ ID NO: 5
MSNEQTFRPETLAIHAGQKPDAETGARAVPIYQTSSYVFRDSEHAANLFGLKEEGF
IYTRIMNPTNDVFEKRIAALEGGIGALALSSGQAAVFYSIINIASAGDEIVSSSSIYGG
TYNLFAHTLRKFGITVKFVDPSDPENFERAITDKTKALFAETIGNPKNDVLDIEAVA
DIAHRHAIPLIVDNTVASPYLLRPIEFGADIVVHSATKFIGGHGNSIGGVIVDSGKFD
WKGSGKFPEFTEPDPSYHGLVYVDAVGEAAYITKARIQLLRDLGAALSPFNAFLLL
QGLETLHLRMQRHSENALAVAKFLEEEEAVESVNYPGLPSHPSHELAKKYLPNGQ
GAIVTFEIKGGVEAGKKLIDSVKLFSHLANIGDSKSLIIHPASTTHEQLSPDEQLSAG
VTPGLVRLSVGTEAIDDILDDLRQAIRQSQTVGVK

SEQ ID NO: 6
MDWKKYGYNTRALHAGYEPPEQATGSRAVPIYQTTSYVFRDSDHAARLFALEEP
GFIYTRIGNPTVSVLEERIAALEEGVGALAVASGQAAITYAILNIAGPGDEIVSGSAL
YGGTYNLFRHTLYKKSGIIVKFVDETDPKNIEEAITEKTKAVYLETIGNPGLTVPDF
EAIAEIAHRHGVPLIVDNTVAPYIFRPFEHGADIVVYSATKFIGGHGTSIGGLIVDSG
KFDWTNGKFPELVEPDPSYHGVSYVETFKEAAYIAKCRTQLLRDLGSCMSPFNAF
LFILGLETLSLRMKKHCENALKIVEFLKSHPAVSWVNYPIAEGNKTRENALKYLKE

GYGAIVTFGVKGGKEAGKKFIDSLTLISHLANIGDARTLAIHPASTTHQQLTEEEQL
KTGVTPDMIRLSVGIEDVEDIIADLDQALRKSQEG

SEQ ID NO: 7
MKLETLAVHAGYSPDPTTRAVAVPIYQTTSYAFDDTQHGADLFDLKVPGNIYTRIM
NPTNDVLEQRVAALEGGVGALAVASGMAAITYAIQTVAEAGDNIVSVAKLYGGTY
NLLAHTLPRIGIQARFAAHDDVAALEALIDERTKAVFCETIGNPAGNIIDLQALADA
AHRHGVPLIVDNTVATPVLCRPFEHGADIVVHSLTKYMGGHGTSIGGIVVDSGKF
DWAANKSRFPLLNTPDPSYHGVTYTEAFGPAAFIGRCRVVPLRNMGAALSPFNAF
LILQGLETLALRMERHCDNALAVARYLQQHPQVAWVKYAGLADNPEHALARRYL
GGRPAAILSFGIQGGSAAGARFIDALKLVVRLVNIGDAKSLACHPASTTHRQLNAE
ELARAGVSDDMVRLSIGIEHIDDILADLDQALAAAAR

SEQ ID NO: 8
MSNNIKAYNNVLELIGETPLIKLSQTVASFPGNYYAKAEAFNPGHSSKDRIALHIIE
QAEKKGILKPGDTIIETTSGNTGFSIAMVSRIKGYDCILAVSSKSSADKIDMLKSMG
AKVYVCPANVSADDPRSYYQVAKRLHDEIKNSIYINQYFNDLNTEAHYLTTGPEI
WNQTAGEITHLVACSGTGGTISGTARFLKEQNPNIKILGVDAYGSVLKKYHETQEF
DKDEIYPYRIEGLGKNLIPTATDFDVIDSFTKVKDEDAAHMARKISQTEGLFVGYT
SGAAMQAVKQLVVEGEFDKNSNVVVIFPDHGSRYMSKIYNDQWMQEQGFVDSK
STANEQHIEYIK

SEQ ID NO: 9
MAVEWTGIANDVTELVGKTPLVYLNKVVDGCVGRIVAKLEFMEPLSSVKDRIGYS
MIADAEEKGLITPGKSVLIEPTSGNTGIGLAFTAAAKGYRLIIVMPASMTLERRMVL
KALGAELVLTDPEKGIRGAFQKVEEIYAKTPNSYVLQQFENPSNPKMHYDTTGPEI
WKATGGKIDALVAGIGTGGTITGSGKFLKEQNPNIKVCAVEPAESPVLVGGQPGSH
GIYGIGAGFIPKVVDVSLLDEVVHVTTSEAIETAKLLAVKEGLFVGVSSGAAAAAA
IKVAKRPEMAGKLIVTIFPSCGERYLSSALFESIREESENMTFEP

SEQ ID NO: 10
MVFPSPLDFFERGKPTPLVRSRLQLPNGVRVWLKLEWYNPFSLSVKDRPAVEIISRL
SRRVEKGSLVADATSSNFGVALSAVARLYGYRARVYLPGAAEEFGKLLPRLLGAQV
IVDPEAPSTVHLLPRVMKDSKNEGFVHVNQFYNDANFEAHMRGTAREIFVQSRRG
GLALRGVAGSLGTSGHMSAAAFYLQSVDPSIRAVLVQPAQGDSIPGIRRVETGML
WINMLDISYTLAEVTLEEAMEAVVEVARSDGLVIGPSGGAAVKALAKKAAEGDLE
PGDYVVVVPDTGFKYLSLVQNALEGAGDSV

SEQ ID NO: 11
MGRFILKCLKCGREYSQEYRLTCENDDSFLRAEYLEKKLELRKQPGIGRFHSWLP
VQEELTTEAGPITYKSEALARELGLSNLYIGFSGYWPEKGAFIKTCSFKELEAHPTM
QLLKESGGKAIVLASAGNTGRAFAHVSALTGTDVYIVVPDSGIPKLWLPEEPTDSI
HLISMTPGNDYTDAINLAGRIAKLPGMVPEGGARNVARREGMGTVMLDAAVTIG
KMPDHYFQAVGSGTGGISAWEASLRLREDGRFGSKLPKLQLTQNLPFVPMYNAW
QEGRRDIIPEIDMKDAKKRIEETYATVLTNRAPPYSVTGGLYDALVDTDGIMYAVS
KEEALDAKALFESLEGIDILPPSAVAAASLLKAVEAGNVGKDDTILLNIAGGGFKRL
KEDFTLFQIEPEITVSNPDVPLEELKL

SEQ ID NO: 12
MTRYDSLLQALGNTPLVGLQRLSPRWDDGRDGPHVRLWAKLEDRNPTGSIKDRPA
VRMIEQAEADGLLRPGATILEPTSGNTGISLAMAARLKGYRLICVMPENTSVERRQ
LLELYGAQIIFSAAEGGSNTAVATAKELAATNPSWVMLYQYGNPANTDSHYCGTGP
ELLADLPEITHFVAGLGTTGTLMGTGRFLREHVANVKIVAAEPRYGEGVYALRNM
DEGFVPELYDPEILTARYSVGAVDAVRRTRELVHTEGIFAGISTGAVLHAALGVGAG
ALAAGERADIALVVADAGWKYLSTGAYAGSLDDAETALEGQLWA

SEQ ID NO: 13

MTVITDITELIGNTPLLRLKNFDVPEGVAVYAKLEMMNPGGSIKDRLGDMLIRDAL
DSGKVKPGGVIIEATAGNTGIGLALSARKYGLKAIFCVPEHFSREKQQIMQALGASI
IHTPRQDGMQGAIQKAIQLETEIENSYCVLQFKNRVNPSTYYKTLGPEMWEALDG
NIHTFVAGAGSGGTFAGTASFLKEKNPAVKTVIVEPVGSILNGGEPHAHKTEGIGM
EFIPDYMDKSHFDEIYTVTDENAFRLVKEAAEKEGLLIGSSSGAALYAALEEAKKA
SAGTNIVTVFPDSSDRYISKQIYEGGI

SEQ ID NO: 14
MVQLHQLFPKHEVFAKLEYMNPGGSMKDRPAKYIIEHGIKHGLITENTHLIESTSG
NLGIALAMIAKIKGLKLTCVVDPKISPTNLKIIKSYGANVEMVEEPDAHGGYLMTR
IAKVQELLATIDDAYWINQYANELNWQSHYHGAGTEIVETIKQPIDYFVAPVSTTG
SIMGMSRKIKEVHPNAQIVAVDAKGSVIFGDKPINRELPGIGASRVPEILNRSEINQV
IHVDDYQSALGCRKLIDYEGIFAGGSTGSIIAAIEQLITSIEEGATIVTILPDRGDRYL
DLVYSDTWLEKMKSRQGVKSE

SEQ ID NO: 15
MTLQYPTIADCVGNTPLVRLQRMAGNTSNTLLLKLEGNNPAGSVKDRPALSMITR
AELRGQIKPGDTLIEATSGNTGIALAMAAAIKGYKMILIMPDNGSAERKAAMTAY
GAELLLVTPEEGMEGARDLAERMAAEGRGQVLDQFANGDNPEAHYTSTGPEIWR
QTQGTITHFVSSMGTTGTIMGNSRYLKEQNPAIQIVGLQPMEGAAIPGIRRWPQEY
LPKIYNAARVDRIIDMAQREAEDTTRRLAREEGIFCGVSSGGAVAGMLRLSAELEN
AVIVAIICDRGDRYLSTGIFDAPN

SEQ ID NO: 16
MSVFSLKIDIADNKFFNGETSPLFSQSQAKLARQFHQKIAGYRPTPLCALDDLANL
FGVKKILVKDESKRFGLNAFKMLGGAYAIAQLLCEKYHLDIETLSFEHLKNAIGEK
MTFATTTDGNHGRGVAWAAQQLGQNAVIYMPKGSAQERVDAILNLGAECIVTDM
NYDDTVRLTMQHAQQHGWEVVQDTAWEGYTKIPTWIMQGYATLADEAVEQMR
EMGVTPTHVLLQAGVGAMAGGVLGYLVDVYSPQNLHSIIVEPDKADCIYRSGVK
GDIVNVGGDMATIMAGLACGEPNPLGWEILRNCATQFISCQDSVAALGMRVLGNP
YGNDPRIISGESGAVGLGVLAAVHYHPQRQSLMEKLALNKDAVVLVISTEGDTDV
KHYREVVWEGKHAVAP

SEQ ID NO: 17
MISAFDIFKIGIGPSSSHTVGPMNAGKSFIDRLESSGLLTATSHIVVDLYGSLSLTGK
GHATDVAIIMGLAGNSPQDVVIDEIPAFIELVTRSGRLPVASGAHIVDFPVAKNIIFHP
EMLPRHENGMRITAWKGQEELLSKTYYSVGGGFIVEEEHFGLSHDVETSVPYDFH
SAGELLKMCDYNGLSISGLMMHNELALRSKAEIDAGFARIWQVMHDGIERGMNT
EGVLPGPLNVPRRAVALRRQLVSSDNISNDPMNVIDWINMYALAVSEENAAGGRV
VTAPTNGACGIIPAVLAYYDKFRRPVNERSIARYFLAAGAIGALYKMNASISGAEV
GCQGEIGVACSMAAAGLTELLGGSPAQVCNAAEIAMEHNLGLTCDPVAGQVQIPC
IERNAINAVKAVNAARMAMRRTSAPRVSLDKVIETMYETGKDMNDKYRETSRGG
LAIKVVCG

SEQ ID NO: 18
MENAKMNSLIAQYPLVKDLVALKETTWFNPGTTSLAEGLPYVGLTEQDVQDAHA
RLSRFAPYLAKAFPETAATGGIIESELVAIPAMQKRLEKEYQQPISGQLLLKKDSHLP
ISGSIKARGGIYEVLAHAEKLALEAGLLTLDDDYSKLLSPEFKQFFSQYSIAVGSTG
NLGLSIGIMSARIGFKVTVHMSADARAWKKAKLRSHGVTVVEYEQDYGVAVEEG
RKAAQSDPNCFFIDDENSRTLFLGYSVAGQRLKAQFAQQGRIVDADNPLFVYLPC
GVGGGPGGVAFGLKLAFGDHVHCFFAEPTHSPCMLLGVHTGLHDQISVQDIGIDN
LTAADGLAVGRASGFVGRAMERLLDGFYTLSDQTMYDMLGWLAQEEGIRLEPSA
LAGMAGPQRVCASVSYQQMHGFSAEQLRNTTHLVWATGGGMVPEEEMNQYLA
KGR

SEQ ID NO: 19
MSIVRLYTARQLPRYRPLRFVRFNSTQVLSPKQRWTELKDTDFIKDSNGEEQPDYV

KLILTSRVYDVVDDAGSPLTYAINLSHRCGTNVYLKREDLLPVFSFKLRGAYNMIA
HLHSNSPTPVPGVIACSAGNHAQGVAFSANKLNIPATIVMPTPTPSIKYTNVSRLGS
QVVLYGDDFDSAKQECARLSAEQNLIDIPPFNHPYVIAGQGTVALEITRQLRLDKL
DAIFVPVGGGGLIAGIAVYLKQIAPHVKIIGVETYDADAMYQSLKNHKSTVLDKV
GLFADGTAVKVLGDETWRLAKDLVDEVVLVTTDELCAAIKDIFEDTRSITEPSGAL
SVAGLKRYIEDHSEIDHKGKTYVPVLSGANMNFDRLRFVSERAVLGEGREVSLAV
TIPEQPGEFAKLQKLINPRAITEFSYRTSGEPTANIFVSFNVIDKKKEKALVINAMTE
AGYKVVDISENELAKSHGRYLVGGKSSIMVMILVKSCVDSISQKDSKKNSNNS

SEQ ID NO: 20
MTDSVTGAGDGTRAVRAGLPEPVKHEPTLPGPVFAAHFHLPGDPTGPYTYGRDE
NPTWTHLERAIGELEAPGRDDVETLVFASGMAAISAVLFSQLSAGDTVVLPDDGY
QVLPLVRAQLEAYGVEVRTAPTGGDAQLDVLDGAKLLWIETPSNPGLDVCDVRRL
VEAAHARGCLVAVDNTLATPLGQRPLELGADFSVASGTKQLTGHGDILLGYVTGG
DAAAMASVRRWRKIVGAIPGPMEAWLAHRSIATLQLRVDRQNANALVIAEALRD
RHEDLGVRYPGLPGDPSHKIASQQMRRFGCVVSFTLPTRARADRFLDALRLVDDA
TSFGGVRSTAERRGRWGGDAVPEGFIRFSAGAEDPQDLVADVLRALNESSQPTPSE

SEQ ID NO: 21
MTASSDHPITTPPRAPSPVHGFGTLAVHAGSPHDPATGAVIEAISLSTTFAQTAVGKP
VGEFEYSRSSNPNRANFEKMVAALEHAKYALAYSSGSATTANILQSLAAGSHVISV
SDVYGGTHRYFTQVAKAHGVKVTFTPEIEVDIRDHITDATKLVWIETPSNPTLRLV
DIRAVATAAHERGILVVVDNTFLSPYVQNPLDHGADIVVHSVTKYINGHSDVVMG
VAAFNSDELYARLSFLQNAIGAVPSAFDSWLAHRGAKTLHLRAREATTNATAIAHA
LEASPLVISVNYPGLESHPHRAIALKQHRNGMGGGMLSFRIHGGHAAAEKFCQYT
KIFTLAESLGGVESLCEIPSSMTHAGIPKAQREAVGIFDDLVRISCGVEDAEDLKAD
VLQALERAVADAANGVSNGVNGTH

SEQ ID NO: 22
MKKKTLMIHGGITGDEKTGAVSVPIYQVSTYKQPKAGQHTGYEYSRTANPTRTAL
EALVTELESGEAGYAFSSGMAAITAVMMLFNSGDHVVLTDDVYGGTYRVMTKVL
NRLGIESTFVDTSSREEVEKAIRPNTKAIYIETPTNPLLKITDLTLMADIAKKAGVLL
IVDNTFNTPYFQQPLTLGADIVLHSATKYLGGHSDVVGGLVVTASKELGEELHFVQ
NSTGGVLGPQDSWLLMRGIKTLGLRMEAIDQNARKIASFLENHPAVQTLYYPGSS
NHPGHELAKTQGAGFGGMISFDIGSEERVDAFLGNLKLFTIAESLGAVESLISVPAR
MTHASIPRERRLELGITDGLIRISVGIEDAEDLLEDIGQALENI

SEQ ID NO: 23
MATVSSCLLRRSRTASRIFKTSLRCFSTTSSSAQTVSGSSPFPFTGTNIKTNVSQLIGR
TPLVYLSKISEGSGAYIAVKQEMMQPTASVKDRPALAMIEDAEKKGLISPGKTVLIE
PTSGNMGISMAFMAAMKGYKMVLTMPSYTSMERRVVMRAFGADLILTDPDKGM
GGTVKKANQLLDSTPDGFMLQQFNNPANTQVHFETTGPEIWEDTQGKVDIFVMG
IGSGGTVSGVGRYLKSQNPNVKIYGVEPAESNILNGGKPGPHLITGNGVGFKPEIL
DMDVMDAVLEVKSDDAVKMARQLALQEGLLVGISSGANTIAALDLAKRPENKG
KLIVTIHPSFGERYLSSALFKELREEAENMQPVPVE

SEQ ID NO: 24
MSGIYPAPVRLESHRQQFPALANKAYFNYGGQGTLPQASLEAIQQAYEYIQRHGPF
SAGVNAWIIEEANKTREAIASELGAPPEAIAITEDVTVGCNIAMWGINWQAGDHL
LLTDCEHPSVIATAQEIARRFDVEVSTCPILSTLNQGDPTAVIAQHLRTQTRLVVLSH
ILWNTGQVLPVKEIVEVCRNYSSSSQTIRVLVDAAQSVGCLPLNLTELGADFYAFT
GHKWWCGPEGIGGLYVRPDAWESLNPTFIGWRGIVSNQAGKPTGWKPNAQRFEV
ATSAYPLYAGVRSAIATHQQWGTPQERYQQICQISEYLWQRLSQLDFVKCLRTSPP
QAGLVSFVLTNEHSHQQLVKSLEQQNLMLRTILDPNCVRACVHYFTQHAEIDKLIE
AMKKIKSKG

MRDSHHNTGFSTRAIHHGYDPLSHGGALVPPVYQTATYAFPTVEYGAACFAGEEP
GHFYSRISNPTLALLEQRMASLEGGEAGLALASGMGAITSTLWTLLRPGDELIVGR
TLYGCTFAFLHHGIGEFGVKVRHVDLNDAKALKAAISSKTRMIYFETPANPNMQL
VDIAAVVEAVRGHDVHVVVDNTYCTPYLQRPLELGADLVVHSATKYLSGHGDIT
AGLVVGRKVLVDRIRLEGLKDMTGAVLSPHDAALLMRGIKTLALRMDRHCANA
QQVAEFLVRQPQVELIHYPGLPSFAQYALAQRQMRLPGGMIAFELKGGIEAGRRF
MNALQLFARAVSLGDAESLAQHPASMTHSSYTPQERAHHGISEGLVRLSVGLEDV
EDLLADVEQALQACC

SEQ ID NO: 26
MTSLKTKVIHGGISTDRTTGAVSVPIYQTSTYKQNGLGKPKEYEYSRSGNPTRHAL
EELIADLEGGVQGFAFSSGLAGIHAVLSLFSAGDHIILADDVYGGTFRLMDKVLTK
TGIIYDLVDLSNLEDLKAAFKAETKAVYFETPSNPLLKVLDIKEISSIAKAHNALTLV
DNTFATPYLQQPIALGADIVLHSATKYLGGHSDVVAGLVTTNSNELASEIGFLQNSI
GAVLGPQDSWLVQRGIKTLALRMEAHSANAQKIAEFLEASQAVSKVYYPGLVNH
EGHEIAKKQMTAFGGMISFELTDENAVKNFVENLRYFTLAESLGGVESLIEVPAVM
THASIPKELREEIGIKDGLIRLSVGVEALEDLLTDLKEALEKE

SEQ ID NO: 27
MPLHNLTRFPRLEFIGAPTPLEYLPRFSDYLGREIFIKRDDVTPMAMGGNKLRKLE
FLAADALREGADTLITAGAIQSNHVRQTAAVAAKLGLHCVALLENPIGTTAENYLT
NGNRLLLDLFNTQIEMCDALTDPNAQLEELATRVEAQGFRPYVIPVGGSNALGAL
GYVESALEIAQQCEGAVNISSVVVASGSAGTHAGLAVGLEHLMPESELIGVTVSRS
VADQLPKVVNLQQAIAKELELTASAEILLWDDYFAPGYGVPNDEGMEAVKLLARL
EGILLDPVYTGKAMAGLIDGISQKRFKDEGPILFIHTGGAPALFAYHPHV

SEQ ID NO: 28
MIFSVDKVRADFPVLSREVNGLPLAYLDSAASAQKPSQVIDAEAEFYRHGYAAVH
RGIHTLSAQATEKMENVRKRASLFINARSAEELVFVRGTTEGINLVANSWGNSNVR
AGDNIIISQMEHHANIVPWQMLCARVGAELRVIPLNPDGTLQLETLPTLFDEKTRL
LAITHVSNVLGTENPLAEMITLAHQHGAKVLVDGAQAVMHHPVDVQALDCDFY
VFSGHKLYGPTGIGILYVKEALLQEMPPWEGGGSMIATVSLSEGTTWTKAPWRFE
AGTPNTGGIIGLGAALEYVSALGLNNIAEYEQNLMHYALSQLESVPDLTLYGPQNR
LGVIAFNLGKHHAYDVGSFLDNYGIAVRTGHHCAMPLMAYYNVPAMCRASLAM
YNTHEEVDRLVTGLQRIHRLLG

SEQ ID NO: 29
MHLARYPRRFIAHLPTPLERLDRLTAELGGPEIWIKRDDCTGLSTGGNKTRKLEFL
MAEAELQGADMVMTQGATQSNHARQTAAFAAKLGMDCHILLEDRTGSNNANY
NNNGNVLLDHLHGATTEKRPGSGLDMNAEMEKVAEKFRADGRKVYTIPGGGSNP
TGALGYVNCAFEMLNQFNERGLKVDHIVHATGSAGTQAGLITGLQAMNAQIPLL
GIGVRAPKPKQEENVYNLACATAEKLGCPGVVAREDVVANTDYVGEGYGIPTESG
LEAIRMFAELEAILLDPVYSAKGAAGFIDLIRKGHFKKGERVVFLHTGGAVALFGY
DNAFDYSGRWVA

SEQ ID NO: 30
MDDYKRILITKILKNEVTEALGCTEVGLIGYAVSLCNISDPFSIEKIELTLNNGSFKN
AYAVGVPNTKKYGILPAVVGGLLGDHKNKLLVFNGIKYSQKLEDFIKERLKIRVINS
PLYCGVKIKDNSGNTFESLIKDNHLNVVIPKINNKLISEINGSEKEEYKNLELLDFLE
YIDEIPEEIIQLVEKTIYTNNNLIKGDFLNFGNDCLSNMVNKTTSACNTRMIGENMP
AMSVAKSGNMGIMATLPIIAYDYSNEQNQEKLIKSILLSVLVTIYATYKSSYLSSMC
GCVSKGGMGAVIGLCYYKNGKNIKKLDSAARTFTANLPGIICDGGKVGCALKLAS
GCFAAYSSLFVDISYENGIVGKNFKECVENISEISKIMGDLDSDIVKIMSKKEI

SEQ ID NO: 31
MANDRSVDWKFSGSEAAKEASAASLGTYSSKLFALCDPLGKPILPPRSKSAETSHT
AEKAVVEAVLCGTGNAYAPSLGLPVARSAVAEYLNRDLPKKLTADDVFMTVGGK

QAIELTVDILAKPKANVLLPKPGFPWDVVRTIYKNLEVRKYDFIPEQNYEIDFDSV
RALVDENTFAIFIINPHNPNGNTYSEVHLKKLAELARELRIMVVSDEVFRWTVFGS
NPFVPMGKFSSIVPVVTLGSISKGWIVPGWRTGWVALHDLDGVFKCTKVLTAAKQ
FLVINSKPPTVIQAALPTILENTPEEFFHKRQKFLKEKADFAYSKLKEIPTLTCYLKP
EACTFLWTELNLSRFADIKDDEEFCEKLATEENLVLLPGIAFGLKNWARHSIDMEIS
TLEDALGRLKSFCERHSIIIEVPLKDVNGVK

## Claims

1. A process for preparing L-glufosinate, comprising steps of:
a) providing a reaction medium, comprising L-homoserine or a salt, an ester, an amide or an anhydride thereof, methylphosphinic acid or a salt or an ester thereof, and an enzyme selected from the group consisting of a transferase which transfers an alkyl other than methyl or an aryl, an ammonia lyase, and a lyase which catalyzes the cleavage of a carbon-sulfur bond;
b) incubating the reaction medium to produce a reaction product comprising L-glufosinate or a phosphonate thereof; and
optionally,
c) recovering the L-glufosinate or the phosphonate thereof from the reaction medium.

2. The process according to claim 1, wherein
the enzyme is selected from the group consisting of enzymes with the EC numbers of EC 2.5.1.-, EC 4.3.1.- and EC 4.4.1.-.

3. The process according to claim 1 or 2, wherein
the reaction medium further comprises phosphopyridoxal or a salt thereof.

4. The process according to any one of claims 1-3, wherein
the enzyme is selected from the group consisting of enzymes with the EC numbers of EC 2.5.1.47, EC 2.5.1.48, EC 2.5.1.49, EC 2.5.1.51, EC 2.5.1.52, EC 2.5.1.65, EC 2.5.1.76, EC 2.5.1.113, EC 2.5.1.134, EC 2.5.1.140, EC 2.5.1.144, EC 4.3.1.15, EC 4.3.1.17, EC 4.3.1.18, EC 4.3.1.19, EC 4.4.1.1, EC 4.4.1.2, EC 4.4.1.9, EC 4.4.1.10, EC 4.4.1.11, EC 4.4.1.13, EC 4.4.1.15, EC 4.4.1.16, EC 4.4.1.25, EC 4.4.1.28 and EC 4.4.1.35.

5. The process according to any one of claims 1-4, wherein
the enzyme is derived from an organism selected from the group consisting of *Thermus thermophiles, Lysinibacillus sphaericus, Clostridioides difficile, Clostridium novyi, Geobacillus stearothermophilus, Thermotoga maritima, Pseudomonas aeruginosa, Nonlabens dokdonensis, Leucaena leucocephala, Aeropyrum pernix, Methanosarcina acetivorans, Mycobacterium tuberculosis, Bacillus subtilis, Staphylococcus aureus, Pseudomonas fluorescens, Escherichia coli, Candida maltosa, Streptomyces phaeochromogenes, Neurospora crassa, Spinacia oleracea, Cyanobacteria bacterium, Pseudomonas putida, Lactococcus lactis, Ruegeria pomeroyi, Methanococcus maripaludis* and *Camelina sativa.*

6. The process according to any one of claims 1-5, wherein
the enzyme is selected from the group consisting of cysteine synthase derived from *Thermus thermophiles,* cystathionine γ-synthase derived from *Lysinibacillus sphaericus,* O-acetyl homoserine aminocarboxypropyltransferase derived from *Clostridioides difficile,* O-acetyl homoserine aminocarboxypropyltransferase derived from *Clostridium novyi,* O-acetyl homoserine aminocarboxypropyltransferase derived from *Geobacillus stearothermophilus,* O-acetyl homoserine aminocarboxypropyltransferase derived from *Thermotoga maritima,* O-acetyl homoserine aminocarboxypropyltransferase derived from *Pseudomonas aeruginosa,* β pyrazolylalanine synthase derived from *Nonlabens dokdonensis,* L-mimosine synthase derived from *Leucaena leucocephala,* O-phosphoserine sulfhydrylase derived from *Aeropyrum pernix,* cysteine synthase derived from *Methanosarcina acetivorans,* [CysO sulfur-carrier protein]-thiocarboxylate-dependent cysteine synthase derived from *Mycobacterium tuberculosis,* cystathionine β synthase derived from *Bacillus subtilis,* N-(2-amino-2-carboxyethyl)-L-glutamate synthase derived from *Staphylococcus aureus,* S-sulfo-L-cysteine synthase derived from *Pseudomonas fluorescens,* diaminopropionate ammonia-lyase derived from *Escherichia coli,* L-serine ammonia-lyase derived from *Escherichia coli,* D-serine ammonia-lyase derived from *Escherichia coli,* threonine ammonia-lyase derived from *Candida maltosa,* cystathionine γ lyase derived from *Streptomyces phaeochromogenes,* cystathionine γ lyase derived from *Neurospora crassa,* homocysteine desulfhydrase derived from *Bacillus subtilis,* L-3-cyanoalanine synthase derived from *Spinacia oleracea,* cysteine lyase derived from *Cyanobacteria bacterium,* methionine γ lyase derived from *Pseudomonas putida,* cysteine-S-conjugate β lyase derived from *Lactococcus lactis,* D-cysteine desulfhydrase derived from *Escherichia coli,* selenocysteine lyase derived from *Escherichia coli,* L-cysteate sulfo-lyase derived from *Ruegeria pomeroyi,* L-cysteine desulfidase derived from *Methanococcus maripaludis* and L-cystine β lyase derived from *Camelina sativa.*

7. The process according to any one of claims 1-6, wherein
the enzyme comprises an amino acid sequence selected from the group consisting of SEQ ID NO: 1-31.

8. The process according to any one of claims 1-7, wherein
the process is a cell-free process.

9. The process according to any one of claims 1-7, wherein
the reaction medium in step a) comprises a host cell expressing the enzyme.

10. The process according to claim 9, wherein
the process further comprises a step of culturing the host cell.

11. The process according to any one of claims 1-10, wherein
in step b), the reaction medium is incubated at 30-40°C, 32-39°C, 34-38°C, 36-38°C or 37°C.
